# FASCICULE DE BREVET EUROPEEN

(11) **EP 0 903 342 B1**
(45) Date de publication et mention de la délivrance du brevet: **17.12.2003**
(21) Numéro de dépôt: 98402149.3
(22) Date de dépôt: 31.08.1998
(51) Int. Cl.: C07D 231/52, C07D 231/28, A61K 7/13

(54) **Composés 4,4-dihydroxypyrazolin-5-ones, leurs procédés de préparation et utilisation cosmétiques**
4,4-Dihydroxypyrazolin-5-on-Derivate, Verfahren zu deren Herstellung und Verwendung als Kosmetika
4,4-Dihydroxypyrazolin-5-one compounds, procedures for their preparation and their use as cosmetics

(30) Priorité: 19.09.1997 FR 9711702
(43) Date de publication de la demande: 24.03.1999
(73) Titulaire: L'OREAL, 75008 Paris (FR)
(72) Inventeur: Vidal, Laurent, 75013 Paris (FR); Malle, Gérard, 77580 Villiers Sur Morin (FR); Garson, Jean-Claude, 92150 Suresnes (FR)
(74) Mandataire: Miszputen, Laurent

(56) Documents cités:
- EP-A- 0 547 864
- EP-A- 0 740 931
- EP-A- 0 796 850
- DE-A- 19 501 304
- FR-A- 2 018 056
- FR-A- 2 746 391
- ALY M F: "X=Y-ZH COMPOUNDS AS POTENTIAL 1,3-DIPOLES. PART 41. AZOMETHINE YLIDE FORMATION FROM THE REACTIONS OF ALPHA-AMINO ACIDS AND ESTERS WITH ALLOXAN (STRECKER DEGRADATION) AND WITH 1-PHENYL-3-METHYLPYRAZOLIN-4, 5-DIONE" TETRAHEDRON, vol. 50, no. 3, 17 janvier 1994, pages 895-906, XP002021233

## Description

L'invention concerne de nouvelles 4,4-dihydroxypyrazolin-5-ones, leurs procédés de préparation et leurs utilisations dans le domaine cosmétique pour colorer la peau et/ou les cheveux mais aussi pour colorer certaines parties de la peau en particulier le visage afin de produire un effet « bonne mine », en rehaussant l'éclat du teint tout en conservant la transparence. L'invention se rapporte également à leurs utilisations dans le domaine cosmétique pour colorer la peau et plus particulièrement en vue de donner à cette dernière un aspect de bronzage.

On sait que la dihydroxyacétone, ou DHA, est un produit particulièrement intéressant qui est couramment utilisé en cosmétique comme agent de bronzage artificiel de la peau ; appliqué sur cette dernière, notamment sur le visage, il permet d'obtenir un effet de bronzage ou de brunissage d'apparence très proche de celui qui peut résulter d'une exposition prolongée au soleil (bronzage naturel) ou sous une lampe UV. Une telle utilisation présente en outre pour avantage d'éviter totalement les risques de réaction cutanée généralement attachés aux expositions prolongées précitées (érythèmes, brûlures, perte d'élasticité, apparition de rides, vieillissement prématuré de la peau, et autres).

Toutefois, l'utilisation de la DHA présente certains inconvénients.

En effet, bien que la couleur produite sur la peau par l'application d'une composition contenant de la DHA soit très proche dé celle obtenue par un bronzage naturel, certains utilisateurs peuvent la juger encore trop jaune.

D'autres inconvénients apparaissent également au cours du stockage des compositions contenant de la DHA. Ainsi, cette dernière présente une fâcheuse tendance, plus ou moins prononcée selon la nature du milieu dans lequel elle est formulée, à se dégrader au cours du temps, cette dégradation se traduisant généralement à terme par un jaunissement non souhaitable des compositions qui la contiennent. Il arrive également qu'au cours du temps de telles compositions présentent une odeur nauséabonde. Enfin, le pH des compositions contenant de la DHA diminue au cours du temps, rendant celles-ci à terme incompatibles avec un emploi en application topique. Ces différents phénomènes font que l'activité de la DHA, et en particulier son aptitude à colorer la peau, est fortement diminuée au moment de l'application de ces compositions sur la peau.

Par ailleurs, l'intensité de la coloration obtenue sur la peau et surtout la rapidité avec laquelle cette coloration se développe sont souvent jugées très insuffisantes par les utilisateurs de produits autobronzants à base de DHA. En effet, la durée nécessaire à l'apparition sur la peau de l'intensité souhaitée est généralement de plusieurs heures.

En vue d'augmenter la rapidité de l'apparition de la couleur due à la DHA, on a cherché à associer cette dernière à d'autres substances. Ainsi, la demande EP-A-547864 propose de fournir la DHA en présence d'un aminoacide et d'une silicone, la DHA et l'aminoacide étant stockés avant application sur la peau dans des compartiments différents. On peut également citer la demande WO-A-9404130 qui décrit un dispositif pour fournir de la DHA en même temps qu'une amine primaire, ces deux composés étant stockés également dans des compartiments différents.

Toutefois, ces dispositifs présentent l'inconvénient d'être compliqués et de ne pas apporter de réelle amélioration quant à la durée d'attente nécessaire pour obtenir une coloration sur la peau satisfaisante. Enfin, ils ne résolvent pas non plus totalement les problèmes dus à la conservation des compositions contenant de la DHA.

En conclusion, il apparaît que la DHA à titre d'agent pour la coloration artificielle de la peau ne donne pas complètement satisfaction, et qu'il existe donc un besoin quant à pouvoir disposer d'autres agents qui, de préférence, ne présentent aucun des désavantages mentionnés ci-dessus.

A cet effet, il est maintenant proposé de nouvelles compositions contenant, dans un support cosmétiquement acceptable, au moins une 4,4-dihydroxypyrazolin-5-one. Le temps nécessaire à la révélation de la couleur après application de ces compositions sur la peau est remarquablement court. Ces compositions présentent en outre une excellente stabilité et confèrent à la peau une couleur très proche de celle conférée par un bronzage naturel. Enfin, la tenue dans le temps de la coloration sur la peau est également remarquable.

Les composés selon l'invention peuvent également être utilisés pour colorer spécifiquement certaines parties de la peau en particulier le visage afin de produire un effet « bonne mine ». Traditionnellement, on a recours à des compositions de maquillage et en particulier des fonds de teint à base de pigments et de charges qui présentent l'inconvénient de faire écran et par suite de trop masquer la peau.

L'utilisation des compositions de l'invention à base des 4,4-dihydroxypyrazolin-5- ones que l'on définiera par la suite permet au contraire de rehausser l'éclat du teint tout en conservant la transparence.

Par ailleurs, ces compositions peuvent trouver une application intéressante dans la coloration capillaire.

L'invention a donc pour premier objet de nouveaux composés 4,4-dihydroxypyrazolin-5-ones répondant à la formule (I) ci-dessous : 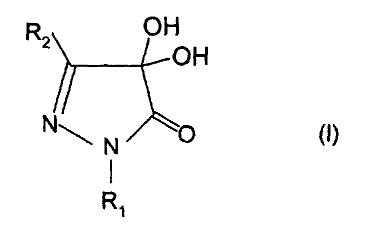 dans laquelle R₁ est choisi parmi :
- un atome d'hydrogène ; un radical alkyle, linéaire ou ramifié, en C₁-C₈,
- un radical -(CH₂)₂-OR' dans lequel R' représente un radical phényle ou naphtyle ;
- un radical -(CH₂)q-R" dans lequel q est égal à 1 ou 2 et R" représente un radical phényle éventuellement substitué par un radical trifluorométhyle ;
- un radical phényle éventuellement substitué par un radical nitro, un radical Cl, un radical alkyle en C₁-C₄ ou un radical alcoxy en C₁-C₄,
et R₂ est choisi parmi :
- un atome d'hydrogène ; un radical alkyle en C₁-C₄, linéaire ou ramifié ; un radical hydroxyalkyle en C₁-C₄ ; un radical méthoxyméthyle ;
- un radical phényle éventuellement substitué par un atome d'halogène, un radical nitro, un radical alkyle en C₁-C₄ ou un radical alcoxy en C₁-C₄ ;
- un radical alcoxy en C₁-C₄ ; un radical trifluorométhyle ; acétamido ; dialkylamino en C₁-C₄ ; carboxy ; méthoxycarbonyle ; éthoxycarbonyle ;
- un radical thiényle, un radical furyle, un radical pyridyle ou un radical pyrazolyle,
- un radical -NHCOR₅ dans lequel R₅ désigne un radical alkyle linéaire ou ramifié en C₁-C₈.

Les conditions ci-dessus pour le choix de R₁ et R₂ étant, de préférence, cumulatives.

Ainsi, parmi les 4,4-dihydroxypyrazolin-5-ones utilisables dans les compositions conformes à l'invention, on peut notamment citer :
- la 1-phényl 4,4-dihydroxypyrazolin-5-one ; la 3-tert-butyl 1-phényl 4,4-dihydroxypyrazolin-5-one ; la 1,3-diphényl 4,4-dihydroxypyrazolin-5-one ; la 1-phényl 3-(4'-méthylphényl) 4,4-dihydroxypyrazolin-5-one ; la 1-phényl 3-(3'-méthoxyméthyl) 4,4-dihydroxypyrazolin-5-one, la 1-phényl 3-(4'-méthoxyméthyl) 4,4-dihydroxypyrazolin-5-one ; la 1-phényl 3-(4'-nitrophényl) 4,4-dihydroxypyrazolin-5-one ; la 3-méthoxy 1-phényl 4,4-dihydroxypyrazolin-5-one ; la 3-éthoxy 1-phényl 4,4-dihydroxypyrazolin-5-one ; la 3-acétamido 1-phényl 4,4-dihydroxypyrazolin-5-one ; la 3-diméthylamino 1-phényl 4,4-dihydroxypyrazolin-5-one ; la 3-diéthylamino 1-phényl 4,4-dihydroxypyrazolin-5-one ; la 3-carboxy 1-phényl 4,4-dihydroxypyrazolin-5-one ; la 3-méthoxycarbonyl 1-phényl 4,4-dihydroxypyrazolin-5-one ; la 3-éthoxycarbonyl 1-phényl 4,4-dihydroxypyrazolin-5-one ;
- la 1-[(3'-trifluorométhyl)benzyl)] 3-méthyl 4,4-dihydroxypyrazolin-5-one ; la 1-[(1'-phényl)éthyl)] 3-méthyl 4,4-dihydroxypyrazolin-5-one ; la 3-méthyl 4,4-dihydroxypyrazolin-5-one ; la 1,3-diméthyl 4,4-dihydroxypyrazolin-5-one ; la 1-(2'-phénoxy)éthyl 3-méthyl 4,4-dihydroxypyrazolin-5-one ; la 1-(2'-naphtyloxy)éthyl 3-propyl 4,4-dihydroxypyrazolin-5-one ; la 1-(2'-naphtyloxy) éthyl 3-hydroxyméthyl 4,4-dihydroxypyrazolin-5-one ; la 3-tert-butyl 1-(2'-phénoxy) éthyl 4,4-dihydroxypyrazolin-5-one ; la 3-méthoxyméthyl 1-(2'-naphtyloxy) éthyl 4,4-dihydroxypyrazolin-5-one ; la 3-méthyl 1-(4'-nitrophényl) 4,4-dihydroxypyrazolin-5-one ; la 3-méthoxy 4,4-dihydroxypyrazolin-5-one ; la 3-éthoxy 4,4-dihydroxypyrazolin-5-one ;
- la 1-méthyl 4,4-dihydroxypyrazolin-5-one ; la 1-méthyl 3-phényl 4,4-dihydroxypyrazolin-5-one ; la 1-méthyl 3-(4'-chlorophényl) 4,4-dihydroxypyrazolin-5-one ; la 1-méthyl 3-(3'-méthoxyphényl) 4,4-dihydroxypyrazolin-5-one ; la 1-méthyl 3-(4'-méthoxyphényl) 4,4-dihydroxypyrazolin-5-one ; la 1-méthyl 3-(3'-nitrophényl) 4,4-dihydroxypyrazolin-5-one ; la 1-méthyl 3-(4'-méthylphényl) 4,4-dihydroxypyrazolin-5-one ; la 1-méthyl 3-(2'-furyl)-4,4-dihydroxypyrazolin-5-one ; la 1-méthyl 3-(2'-thienyl)-4,4-dihydroxypyrazolin-5-one ; la 1-méthyl 3-(5'-pyrazolyl) 4,4-dihydroxypyrazolin-5-one ; la 1-méthyl 3-(4'-pyridyl) 4,4-dihydroxypyrazolin-5-one ; la 1-méthyl 3-méthoxy 4,4-dihydroxypyrazolin-5-one ; la 3-éthoxy 1-méthyl 4,4-dihydroxypyrazolin-5-one ; la 3-diméthylamino 1-méthyl 4,4-dihydroxypyrazolin-5-one ; la 3-diéthylamino 1-méthyl 4,4-dihydroxypyrazolin-5-one ; la 3-acétamido 1-méthyl 4,4-dihydroxypyrazolin-5-one ; la 3-carboxy 1-méthyl 4,4-dihydroxypyrazolin-5-one ; la 3-méthoxycarbonyle 1-méthyl 4,4-dihydroxypyrazolin-5-one ; la 3-éthoxycarbonyle 1-méthyl 4,4-dihydroxypyrazolin-5-one ;
- la 1-éthyl 4,4-dihydroxypyrazolin-5-one ; la 1-méthyl 3-méthyl 4,4-dihydroxypyrazolin-5-one ; la 1-éthyl 3-phényl 4,4-dihydroxypyrazolin-5-one ; la 1-éthyl 3-(4'-chlorophényl) 4,4-dihydroxypyrazolin-5-one ; la 1-éthyl 3-(3'méthoxyphényl) 4,4-dihydroxypyrazolin-5-one ; la 1-éthyl 3-(4'-méthoxyphényl) 4,4-dihydroxypyrazolin-5-one ; la 1-éthyl 3-(3'-nitrophényl) 4,4-dihydroxypyrazolin-5-one ; la 1-éthyl 3-(4'-méthylphényl) 4,4-dihydroxypyrazolin-5-one ; la 1-éthyl 3-(2'-furyl) 4,4-dihydroxypyrazolin-5-one ; la 1-éthyl 3-(2'-thiényl) 4,4-dihydroxypyrazolin-5-one ; la 1-éthyl 3-(5'-pyrazolyl) 4,4-dihydroxypyrazolin-5-one ; la 1-éthyl 3-méthoxy 4,4-dihydroxypyrazolin-5-one ; la 1-éthyl 3-éthoxy 4,4-dihydroxypyrazolin-5-one ; la 1-éthyl 3-diméthylamino 4,4-dihydroxypyrazolin-5-one ; la 1-éthyl 3-diéthylamino 4,4-dihydroxypyrazolin-5-one ; la 1-éthyl 3-acétamido 4,4-dihydroxypyrazolin-5-one ; la 1-éthyl 3-carboxy 4,4-dihydroxypyrazolin-5-one ; la 1-éthyl 3-méthoxycarbonyl 4,4-dihydroxypyrazolin-5-one ; la 1-éthyl 3-éthoxycarbonyl 4,4-dihydroxypyrazolin-5-one ;
- la 1-isopropyl 4,4-dihydroxypyrazolin-5-one ; la 1-isopropyl 3-méthyl 4,4-dihydroxypyrazolin-5-one ; la 1-isopropyl 3-phényl 4,4-dihydroxypyrazolin-5-one ; la 1-isopropyl 3-(4'-chlorophényl) 4,4-dihydroxypyrazolin-5-one ; la 1-isopropyl 3-(3'-méthoxyphényl) 4,4-dihydroxypyrazolin-5-one ; la 1-isopropyl 3-(4'-méthoxyphényl) 4,4-dihydroxypyrazolin-5-one ; la 1-isopropyl 3-(3'-nitrophényl) 4,4-dihydroxypyrazolin-5-one ; la 1-isopropyl 3-(4'-méthylphényl) 4,4-dihydroxypyrazolin-5-one ; la 1-sopropyl 3-(2'-furyl) 4,4-dihydroxypyrazolin-5-one ; la 1-isopropyl 3-(2'-thiényl) 4,4-dihydroxypyrazolin-5-one ; la 1-isopropyl 3-(5'-pyrazolyl) 4,4-dihydroxypyrazolin-5-one ; la 1-isopropyl 3-méthoxy 4,4-dihydroxypyrazolin-5-one ; la 1-isopropyl 3-éthoxy 4,4-dihydroxypyrazolin-5-one ; la 1-isopropyl 3-diméthylamino 4,4-dihydroxypyrazolin-5-one ; la 1-isopropyl 3-diéthylamino 4,4-dihydroxypyrazolin-5-one ; la 1-isopropyl 3-acétamido 4,4-dihydroxypyrazolin-5-one ; la 1-isopropyl 3-carboxy 4,4-dihydroxypyrazolin-5-one ; la 1-isopropyl 3-méthoxycarbonyl 4,4-dihydroxypyrazolin-5-one ; la 1-isopropyl 3-éthoxycarbonyl 4,4-dihydroxypyrazolin-5-one ;
- la 1-tert-butyl 4,4-dihydroxypyrazolin-5-one ; la 1-tert-butyl 3-méthyl 4,4-dihydroxypyrazolin-5-one ; la 1-tert-butyl 3-phényl 4,4-dihydroxypyrazolin-5-one ; la 1-tert-butyl 3-(4'-chlorophényl) 4,4-dihydroxypyrazolin-5-one ; la 1-tert-butyl 3-(3'-méthoxyphényl) 4,4-dihydroxypyrazolin-5-one ; la 1-tert-butyl 3-(4'-méthoxyphényl) 4,4-dihydroxypyrazolin-5-one ; la 1-tert-butyl 3-(3'-nitrophényl) 4,4-dihydroxypyrazolin-5-one ; la 1-tert-butyl 3-(4'-méthylphényl) 4,4-dihydroxypyrazolin-5-one ; la 1-tert-butyl 3-(2'-furyl) 4,4-dihydroxypyrazolin-5-one ; la 1-tert-butyl 3-(2'-thiényl) 4,4-dihydroxypyrazolin-5-one ; la 1-tert-butyl 3-(5'-pyrazolyl) 4,4-dihydroxypyrazolin-5-one ; la 1-tert-butyl 3-méthoxy 4,4-dihydroxypyrazolin-5-one ; la 1-tert-butyl 3-éthoxy 4,4-dihydroxypyrazolin-5-one ; la 1-tert-butyl 3-diméthylamino 4,4-dihydroxypyrazolin-5-one ; la 1-tert-butyl 3-diéthylamino 4,4-dihydroxypyrazolin-5-one ; la 1-tert-butyl 3-acétamido 4,4-dihydroxypyrazolin-5-one ; la 1-tert-butyl 3-carboxy 4,4-dihydroxypyrazolin-5-one la 1-tert-butyl 3-méthoxycarbonyl 4,4-dihydroxypyrazolin-5-one ; la 1-tert-butyl 3-éthoxycarbonyl 4,4-dihydroxypyrazolin-5-one ;
- la 1-octyl 4,4-dihydroxypyrazolin-5-one ; la 1-octyl 3-méthyl 4,4-dihydroxypyrazolin-5-one ; la 1-octyl 3-phényl 4,4-dihydroxypyrazolin-5-one ; la 1-octyl 3-(4'-chlorophényl) 4,4-dihydroxypyrazolin-5-one ; la 1-octyl 3-(3'-méthoxyphényl) 4,4-dihydroxypyrazolin-5-one ; la 1-octyl 3-(4'-méthoxyphényl) 4,4-dihydroxypyrazolin-5-one ; la 1-octyl 3-(3'-nitrophényl) 4,4-dihydroxypyrazolin-5-one ;
- la 1-(4'-méthylphényl) 4,4-dihydroxypyrazolin-5-one ; la 1-(4'-méthylphényl) 3-méthyl 4,4-dihydroxypyrazolin-5-one ; la 1-(4'-méthylphényl) 3 phényl 4,4-dihydroxypyrazolin-5-one ; la 1-(4'-méthylphényl) 3-(4'-chlorophényl) 4,4-dihydroxypyrazolin-5-one ; la 1-(4'-méthylphényl) 3-(3'-méthoxyphényl) 4,4-dihydroxypyrazolin-5-one ; la 1-(4'-méthylphényl) 3-(4'-méthoxyphényl) 4,4-dihydroxypyrazolin-5-one ; la 1-(4'-méthylphényl) 3-(3'-nitrophényl) 4,4-dihydroxypyrazolin-5-one ; la 1-(4'-méthylphényl) 3-(4'-méthylphényl) 4,4-dihydroxypyrazolin-5-one ; la 1-(4'-méthylphényl) 3-(2'-furyl) 4,4-dihydroxypyrazolin-5-one ; 1-(4'-méthylphényl) 3-(2'-thiényl) 4,4-dihydroxypyrazolin-5-one ; la 1-(4'-méthylphényl) 3-(5'-pyrazolyl) 4,4-dihydroxypyrazolin-5-one ; la 1-(4'-méthylphényl) 3-méthoxy 4,4-dihydroxypyrazolin-5-one ; la 1-(4'-méthylphényl) 3-éthoxy 4,4-dihydroxypyrazolin-5-one ; la 1-(4'-méthylphényl) 3-diméthylamino 4,4-dihydroxypyrazolin-5-one ; la 1-(4'-méthylphényl) 3-diéthylamino 4,4-dihydroxypyrazolin-5-one ; la 1-(4'-méthylphényl) 3-acétamido 4,4-dihydroxypyrazolin-5-one ; la 1-(4'-méthylphényl) 3-carboxy 4,4-dihydroxypyrazolin-5-one ; la 1-(4'-méthylphényl) 3-méthoxycarbonyl 4,4-dihydroxypyrazolin-5 one ; la 1-(4'-méthylphényl) 3-éthoxycarbonyl 4,4-dihydroxypyrazolin-5-one ;
- la 1-benzyl 4,4-dihydroxypyrazolin-5-one ; la 1-benzyl 3-méthyl 4,4-dihydroxypyrazolin-5-one ; la 1-benzyl 3-phényl 4,4-dihydroxypyrazolin-5-one ; la 1-benzyl 3-(4'méthylphényl) 4,4-dihydroxypyrazolin-5-one ; la 1-benzyl 3-(3'-méthoxyphényl) 4,4-dihydroxypyrazolin-5-one ; la 1-benzyl 3-(4'-méthoxyphényl) 4,4-dihydroxypyrazolin-5-one ; la 1-benzyl 3-(3'-nitrophényl) 4,4-dihydroxypyrazolin-5-one ;
- la 1-(4'-méthoxyphényl) 4,4-dihydroxypyrazolin-5-one ; la 1-(4'-méthoxyphényl) 3-méthyl 4,4-dihydroxypyrazolin-5-one ; la 1-(4'-méthoxyphényl) 3-(3'-méthoxyphényl) 4,4-dihydroxypyrazolin-5-one ; la 1-(4'-méthoxyphényl) 3-phényl 4,4-dihydroxypyrazolin-5-one ; la 1-(4'-méthoxyphényl) 3-(4'-chlorophényl) 4,4-dihydroxypyrazolin-5-one ; la 1-(4'-méthoxyphényl) 3-(4'-méthoxyphényl) 4,4-dihydroxypyrazolin-5-one ; la 1-(4'-méthoxyphényl) 3-(3'-nitrophényl) 4,4-dihydroxypyrazolin-5-one ; la 1-(4'-méthoxyphényl) 3-méthoxy 4,4-dihydroxypyrazolin-5-one ; la 1-(4'-méthoxyphényl) 3-éthoxy 4,4-dihydroxypyrazolin-5-one ; la 1-(4'-méthoxyphényl) 3-diméthylamino 4,4-dihydroxypyrazolin-5-one ; la 1-(4'-méthoxyphényl) 3-diéthylamino 4,4-dihydroxypyrazolin-5-one ; la 1-(4'-méthoxyphényl) 3-acétamido 4,4-dihydroxypyrazolin-5-one ; la 1-(4'-méthoxyphényl) 3-carboxy 4,4-dihydroxypyrazolin-5-one ; la 1-(4'-méthoxyphényl) 3-méthoxycarbonyl 4,4-dihydroxypyrazolin-5-one ; la 1-(4'-méthoxyphényl) 3-éthoxycarbonyl 4,4-dihydroxypyrazolin-5-one;
- la 1-(4'chlorophényl) 4,4-dihydroxypyrazolin-5-one ; la 1-(4'-chlorophényl) 3-méthyl 4,4-dihydroxypyrazolin-5-one ; la 1-(4'-chlorophényl) 3-phényl 4,4-dihydroxypyrazolin-5-one ; la 1-(4'-chlorophényl) 3-(4'-méthylphényl) 4,4-dihydroxypyrazolin-5-one ; la 1-(4'-chlorophényl) 3-(3'-méthoxyphényl) 4,4-dihydroxypyrazolin-5-one ; la 1-(4'-chlorophényl) 3-(4'-méthoxyphényl) 4,4-dihydroxypyrazolin-5-one ; la 1-(4'-chlorophényl) 3-(3'-nitrophényl) 4,4-dihydroxypyrazolin-5-one ; la 1-(4'-chlorophényl) 3-méthoxy 4,4-dihydroxypyrazolin-5-one ; la 1-(4'-chlorophényl) 3-éthoxy 4,4-dihydroxypyrazolin-5-one ; la 1-(4'-chlorophényl) 3-diméthylamino 4,4-dihydroxypyrazolin-5-one ; la 1-(4'-chlorophényl) 3-diéthylamino 4,4-dihydroxypyrazolin-5-one ; la 1-(4'-chlorophényl) 3-acétamido 4,4-dihydroxypyrazolin-5-one ; la 1-(4'-chlorophényl) 3-carboxy 4,4-dihydroxy-pyrazolin-5-one ; la 1-(4'-chloro-phényl) 3-méthoxycarbonyl 4,4-dihydroxypyrazolin-5-one ; la 1-(4'-chlorophényl) 3-éthoxycarbonyl 4,4-dihydroxypyrazolin-5-one ;
- la 1-(4'-nitrophényl) 4,4-dihydroxypyrazolin-5-one ; la 1-(4'-nitrophényl) 3-méthyl 4,4-dihydroxypyrazolin-5-one ; la 1-(4'-nitrophényl) 3-phényl 4,4-dihydroxypyrazolin-5-one ; la 1-(4'-nitrophényl) 3-(4'-méthylphényl) 4,4-dihydroxypyrazolin-5-one ; la 1-(4'-nitrophényl) 3-(3'-méthoxyphényl) 4,4-dihydroxypyrazolin-5-one ; la 1-(4'-nitrophényl) 3-(4'-méthoxyphényl) 4,4-dihydroxypyrazolin-5-one ; la 1-(4'-nitrophényl) 3-(3'-nitrophényl) 4,4-dihydroxypyrazolin-5-one ;
- la 1-phényl 3-trifluorométhyl 4,4-dihydroxypyrazolin-5-one ; la 1-méthyl 3-trifluorométhyl 4,4-dihydroxypyrazolin-5-one ; la 1-isopropyl 3-trifluorométhyl 4,4-dihydroxypyrazolin-5-one ; la 1-éthyl 3-trifluorométhyl 4,4-dihydroxypyrazolin-5-one ; la 3-trifluorométhyl 4,4-dihydroxypyrazolin-5-one.

Parmi les 4,4-dihydroxypyrazolin-5-ones utilisables dans le cadre de la présente invention, on préfère tout particulièrement celles pour lesquelles, cumulativement, R₁ est choisi parmi :
- l'hydrogène et les radicaux méthyle, éthyle, isopropyle, tertiobutyle ou phényle substitué ou non ;
et R₂ est choisi parmi :
- l'hydrogène ou les radicaux méthyle, phényle, méthoxyphényle, méthoxy, éthoxy, carboxy, méthoxycarbonyle, éthoxycarbonyle, acétamido, diméthylamino, diéthylamino, trifluorométhyle, furyle, pyridyle.

On préfère plus particulièrement utiliser la 3-méthyl 4,4-dihydroxypyrazolin-5-one ; la 1,3-diméthyl 4,4-dihydroxypyrazolin-5-one ; la 1-éthyl 3-méthyl 4,4-dihydroxypyrazolin-5-one ; la 1-isopropyl 3-méthyl 4,4-dihydroxypyrazolin-5-one ; la 1-tertbutyl 3-méthyl 4,4-dihydroxypyrazolin-5-one ; la 1-méthyl 3-phényl 4,4-dihydroxypyrazolin-5-one ; la 1-méthyl 3-(3'-méthoxyphényl) 4,4-dihydroxypyrazolin-5-one ; la 3(2'-furyl) 1-méthyl 4,4-dihydroxypyrazolin-5-one ; la 1-méthyl 3-(4'-pyridyl) 4,4-dihydroxypyrazolin-5-one ; la 1-méthyl 3-méthoxy 4,4-dihydroxypyrazolin-5-one ; la 3-éthoxy 1-méthyl 4,4-dihydroxypyrazolin-5-one ; la 3-diméthylamino 1-méthyl 4,4-dihydroxypyrazolin-5-one ; la 3-diéthylamino 1-méthyl 4,4-dihydroxypyrazolin-5-one ; la 3-acétamido 1-méthyl 4,4-dihydroxypyrazolin-5-one ; la 1-phényl 4,4-dihydroxypyrazolin-5-one ; la 1-méthyl 4,4-dihydroxypyrazolin-5-one ; la 1-éthyl 4,4-dihydroxypyrazolin-5-one ; la 1-isopropyl 4,4-dihydroxypyrazolin-5-one ; la 1-tertbutyl 4,4-dihydroxypyrazolin-5-one ; la 3-méthoxy 1-phényl 4,4-dihydroxypyrazolin-5-one ; la 3-éthoxy 1-phényl 4,4-dihydroxypyrazolin-5-one ; la 3-acétamido 1-phényl 4,4-dihydroxypyrazolin-5-one ; la 3-diméthylamino 1-phényl 4,4-dihydroxypyrazolin-5-one ; la 3-diéthylamino 1-phényl 4,4-dihydroxypyrazolin-5-one ; la 1-phényl 3-trifluorométhyl 4,4-dihydroxypyrazolin-5-one ; la 1-méthyl 3-trifluorométhyl 4,4-dihydroxypyrazolin-5-one ; la 1-isopropyl 3-trifluorométhyl 4,4-dihydroxypyrazolin-5-one ; la 1-éthyl 3-trifluorométhyl 4,4-dihydroxypyrazolin-5-one ; la 3-trifluorométhyl 4,4-dihydroxypyrazolin-5-one ; la 3-carboxy 1-phényl 4,4-dihydroxypyrazolin-5-one ; la 3-méthoxycarbonyl 1-phényl 4,4-dihydroxypyrazolin-5-one ; la 3-éthoxycarbonyl 1-phényl 4,4-dihydroxypyrazolin-5-one ; la 3-méthoxy 4,4-dihydroxypyrazolin-5-one ; la 3-méthoxy 4,4-dihydroxypyrazolin-5-one ; la 3-carboxy 1-méthyl 4,4-dihydroxypyrazolin-5-one ; la 3-méthoxycarbonyle 1-méthyl 4,4-dihydroxypyrazolin-5-one ; la 3-éthoxycarbonyle 1-méthyl 4,4-dihydroxypyrazolin-5-one.

L'invention a également pour objet des compositions cosmétiques ou dermatologiques comprenant, dans un support cosmétiquement acceptable, au moins une 4,4-dihydroxypyrazolin-5-one répondant à la formule (I) ci-dessus.

Dans une forme préférée de réalisation de l'invention, ces compositions sont destinées au bronzage artificiel de la peau.

Ces nouvelles compositions présentent l'avantage de conférer à la peau un bronzage très proche du bronzage naturel et ce, en un temps extrêmement court. En effet, quelques minutes suffisent pour obtenir un teint bronzé.

La présente invention a encore pour objet un procédé de traitement cosmétique de la peau destiné à la bronzer et/ou la brunir artificiellement, consistant à appliquer sur celle-ci une quantité efficace d'une composition cosmétique conforme à l'invention ou d'une 4,4-dihydroxypyrazolin-5-one telle que mentionnée ci-dessus.

La présente invention a aussi pour objet l'utilisation d'une 4,4-dihydroxypyrazolin-5-one telle que mentionnée ci-dessus dans, ou pour la fabrication de, compositions destinées au bronzage et/ou au brunissage artificiels de la peau.

Dans une autre forme de réalisation de l'invention, ces compositions cosmétiques sont destinées pour le maquillage et plus particulièrement pour colorer la peau, en particulier le visage pour lui donner un aspect « bonne mine» en rehaussant l'éclat du teint.

La présente invention a encore pour objet un procédé de maquillage de la peau, en particulier du visage destiné à lui donner un aspect « bonne mine» en rehaussant l'éclat du teint, consistant à appliquer sur la peau une quantité efficace d'une composition cosmétique conforme à l'invention ou d'une 4,4-dihydroxypyrazolin-5-one telle que mentionnée ci-dessus.

La présente invention a aussi pour objet l'utilisation d'une 4,4-dihydroxypyrazolin-5-one telle que mentionnée ci-dessus dans, ou pour la fabrication de, compositions de maquillage de la peau, en particulier du visage destiné à lui donner un aspect « bonne mine» en rehaussant l'éclat du teint.

Dans une autre forme de réalisation de l'invention, ces compositions cosmétiques sont destinées à colorer les cheveux.

La présente invention a ainsi également pour objet un procédé de traitement cosmétique des cheveux destiné à les colorer, consistant à appliquer sur les cheveux une quantité efficace d'une composition conforme à l'invention ou d'une 4,4-dihydroxypyrazolin-5-one telle que mentionnée ci-dessus.

La présente invention a encore pour objet l'utilisation d'une 4,4-dihydroxypyrazolin-5-one telle que mentionnée ci-dessus dans, ou pour la fabrication de, compositions destinées à colorer les cheveux.

D'autres caractéristiques, aspects et avantages de la présente invention apparaîtront à la lecture de la description détaillée qui va suivre.

La ou les 4,4-dihydroxypyrazolin-5-ones sont utilisées en une quantité efficace pour conférer une coloration soit sur la peau soit sur les cheveux, selon les cas. Ainsi, elles sont généralement présentes dans les compositions selon l'invention à une concentration comprise entre 0,05 et 10 % en poids, de préférence entre 0,05 et 5 % en poids, par rapport au poids total de la composition.

Lorsque les compositions selon l'invention sont appliquées sur la peau, le pH peut varier entre 3 et 10. Il est de préférence compris entre 3 et 7.

Lorsque les compositions selon l'invention sont destinées à la coloration des cheveux, le pH peut varier entre 2 et 8. Il est de préférence compris entre 2 et 6.

Le véhicule (ou support cosmétiquement acceptable) des compositions selon l'invention est de préférence l'eau, seule ou avec un ou plusieurs solvants organiques ou corps gras.

Les solvants utilisables sont de préférence choisis parmi les alcools tels que l'alcool éthylique ou l'alcool cétylique, ou encore parmi le propylène glycol, l'éthylène glycol monoéthyléther et l'éthylène glycol monobutyléther

Comme corps gras, on peut citer les huiles et les cires d'origine minérale, animale ou végétale.

Par huile, on entend un composé liquide à température ambiante. Par cire, on entend un composé solide ou substantiellement solide à température ambiante, et dont le point de fusion est généralement supérieur à 35°C.

Comme huiles, on peut citer les huiles minérales (vaseline); végétales (huile d'amande douce, de macadamia, de pépin de cassis) ; synthétiques comme le perhydrosqualène, les alcools, les acides ou les esters gras (palmitate d'octyle, lanolate d'isopropyle, les triglycérides dont ceux des acides caprique/caprylique), les esters et éthers gras oxyéthylénés ou oxypropylénés; siliconées (cyclométhicone, polydiméthysiloxanes ou PDMS) ou fluorées, les polyalkylènes.

Comme composés cireux, on peut citer l'huile de jojoba, la paraffine, la cire de carnauba, la cire d'abeille, l'huile de ricin hydrogénée.

Les autres constituants pouvant rentrer dans la formulation des compositions visées par l'invention, en particulier les épaississants, les émulsionnants, les gélifiants sont ceux qui sont classiquement utilisés dans le domaine cosmétique et/ou dermatologique.

Comme émulsionnants, on peut utiliser les esters d'acides gras et de polyéthylène glycol (PEG), les esters d'acides gras et de glycérol (stéarate de glycéryle) ou les esters d'acides gras et de sucre (stéarate de sorbitane), ainsi que leurs dérivés polyoxyéthylénés ou polyoxypropylénés, les cyclométhicones et diméthicones copolyols, les tensioactifs anioniques (alkylphosphate de K ou de Na), les alcools gras polyalcoxylés.

De préférence, on utilise les alcools gras polyalcoxylés tels que les alcools butyliques oxypropylénés, les alcools capryliques oxyéthylénés, les alcools cétyliques oxyéthylénés.

Comme épaississants, on peut utiliser les acides polyacryliques réticulés, les gommes de guar et celluloses modifiées ou non telles que la gomme de guar hydroxypropylée, la méthylhydroxyéthylcellulose, l'hydroxypropylméthyl cellulose ou encore l'hydroxyéthylcellulose.

Comme gélifiants, on peut citer les argiles modifiées (bentones), les sels métalliques d'acides gras (stéarate d'aluminium), les copolymères éthylène/acrylate, les silices, les polyéthylènes, les silicates de calcium ou encore l'éthylcellulose.

Les compositions selon l'invention peuvent en outre contenir tout autre constituant cosmétiquement ou dermatologiquement acceptable habituellement utilisé dans ce genre de compositions, tels que les adoucissants, les antioxydants, les opacifiants, les stabilisants, les émollients, les agents répulsifs contre les insectes, les filtres solaires organiques actifs dans l'UV-A et/ou l'UV-B, les pigments et les nanopigments minéraux photoprotecteurs, les agents hydratants, les vitamines, les parfums, les conservateurs, les charges, les séquestrants, les colorants, et en particulier les composés connus pour leur action auto-bronzante comme par exemple la dihydroxyacétone, le méthylglycoxal, l'aldéhyde glycérique, l'érythrulose, l'alloxane, le dialdéhyde 2,3-dihydroxysuccinique, le dialdéhyde 2-amino-3-hydroxysuccinique, le dialdéhyde 2-benzylamino-3-hydroxysuccinique.

Bien entendu, l'homme de l'art veillera à choisir ce ou ces éventuels composés complémentaires et/ou leurs quantités de manière telle que les propriétés avantageuses attachées intrinsèquement à la composition conforme à l'invention ne soient pas, ou substantiellement pas, altérées par la ou les adjonctions envisagées.

Cette composition peut se présenter en particulier sous forme d'émulsion, simple ou complexe (H/E, E/H, H/E/H ou E/H/E) telle qu'une crème, un lait, un gel ou un gel crème, de poudre, de bâtonnet solide et éventuellement être conditionnée en aérosol et se présenter sous forme de mousse ou de spray.

De préférence, cette composition se présente sous la forme d'une émulsion huile-dans-eau.

Les compositions concernées par l'invention peuvent être préparées selon les techniques bien connues de l'homme de l'art, en particulier celles destinées à la préparation d'émulsions de type huile-dans-eau ou eau-dans-huile.

L'invention a également pour objet un procédé de préparation des composés de formule (I), ce procédé étant caractérisé par le fait qu'il comprend les étapes suivantes (les significations de R₁ et R₂ sont telles que données ci-avant) :
(i) on fait réagir une hydrazine monosubstituée R₁NH-NH₂ avec un β-cétoester de structure : 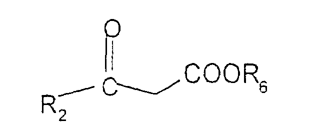 dans laquelle R₆ désigne un radical alkyle linéaire ou ramifié en C₁-C₄, de préférence en milieu alcoolique tel que dans le méthanol, éthanol ou isopropanol, à une température comprise entre 65 et 85°C, de préférence au reflux du solvant utilisé de façon à obtenir une pyrazolin-5-one ① qu'on fait ensuite réagir avec un composé nitroso-aromatique ② de façon à obtenir la 4-arylimino pyrazolin-5-one correspondante ③ : 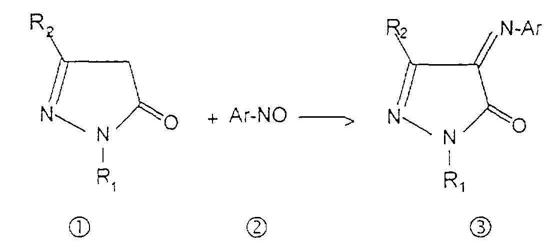 cette réaction étant conduite de préférence dans un alcool inférieur tel que le méthanol, l'éthanol ou l'isopropanol à une température comprise entre 65 °C et 85 °C, au reflux du solvant utilisé, et de préférence en présence d'une base faible de type carbonate ou bicarbonate en quantité catalytique,
(ii) puis on hydrolyse la 4-arylimino pyrazolin-5-one ③, de préférence en milieu acide dilué fort, pour obtenir le dérivé de 4,4-dihydroxypyrazolin-4,5one correspondant de formule (I) :

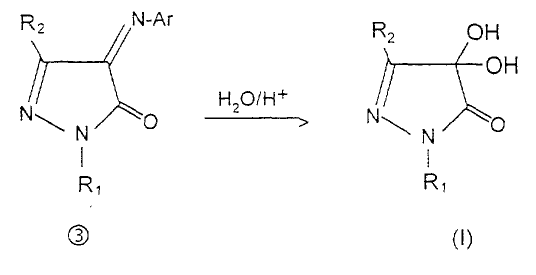

Dans un tel procédé, le dérivé nitroso aromatique de la première étape est de préférence une p-nitrosodialkylaniline de formule ② : 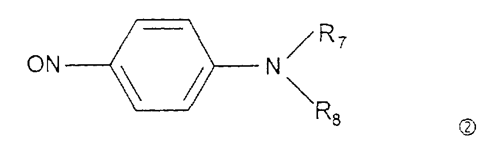 dans laquelle R₇ et R₈ représentent un radical alkyle en C₁-C₄, linéaire ou ramifié.

L'hydrolyse acide de la deuxième étape du procédé de préparation selon l'invention est réalisée de préférence avec de l'acide sulfurique dilué ou de l'acide chlorhydrique aqueux à température ambiante en présence d'un co-solvant de la 4,4-dihydroxypyrazolin-5-one, non miscible à l'eau, ce qui permet d'extraire avantageusement le composé au fur et à mesure de sa formation facilitant son isolement avec une très grande pureté. Le co-solvant non miscible à l'eau peut être un solvant halogéné tel que par exemple le dichlorométhane ou le 1,2-dichloroéthane. Dans une forme préférée de réalisation de l'invention, le co-solvant non miscible à l'eau est un éther tel que l'éther diéthylique ou diisopropylique.

Des exemples concrets, mais nullement limitatifs, destinés à illustrer l'invention vont maintenant être donnés.

### EXEMPLE DE PREPARATION : Synthèse de la 3-acétamido-4,4-dihydroxy 1-phényl pyrazolin-5-one

### Etape 1 : Préparation du 3-acétamido-5-hydroxy-1-phényl-pyrazole :

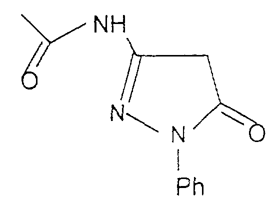

A une solution de 100 g (0,571 mole) de 3-amino-1-phényl-pyrazolin-5-one dans 2 litres d'acide acétique ont été ajoutés goutte à goutte 55 cm3 d'anhydride acétique. La solution a été portée au reflux pendant 4 heures 30 puis refroidie à environ 17°C. Le précipité a été essoré sur verre fritté N°4 et lavé par de l'éther diisopropylique. Le filtrat a été concentré puis glacé. Un deuxième jet cristallisé s'est formé, a été essoré, lavé par de l'éther diisopropylique et séché sous vide à 40°C avec le premier jet. Après recristallisation dans l'éthanol, on a isolé 76,24 g de poudre beige. Les spectres RMN¹H et RMN¹³C du produit obtenu sont conformes à la structure attendue.

### Etape 2 : Préparation du 3-acétamido-4-(N-(4'-diméthylaminophényl) imino-1-phényl-pyrazolin-5-one :

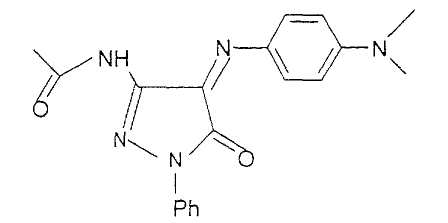

A une solution de 75,84 g (0,349 mole) de 3-acétamido-5-hydroxy-1-phényl-pyrazole dans 5 litres de propan-1-ol tiédie à 45°C a été ajoutée une solution de 52,43 g (0,349 mole) de N,N-diméthyl-4-nitrosoaniline dans 1 litre de propan-1-ol, puis 5g de carbonate de potassium (0,035 mole). Le mélange réactionnel a été porté au reflux pendant 4 heures. Le propan-1-ol a été ensuite distillé au 3/4 puis la solution glacée. Le produit a cristallisé, a été essoré et lavé par du cyclohexane sur verre fritté N°4. Après séchage sous vide à 40°C, on a obtenu 80,48 g d'une poudre noire. Les spectres RMN ¹H et RMN ¹³C du produit obtenu sont conformes à la structure attendue.

### Etape 3 : Préparation du 3-acétamido-1-phényl-4,4-dihydroxypyrazolin-5-one de formule (I) :

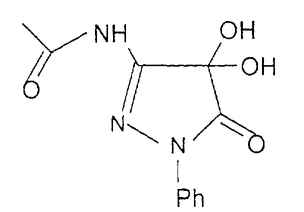

A une solution de 65 g (0,186 mole) de 3-acétamido-4-(N-(4'-diméthylaminophényl) imino-1-phényl-pyrazolin-5-one dans 3,4 litres de THF ont été ajoutés goutte à goutte 600 ml d'acide sulfurique 2N. Le mélange réactionnel a été agité à température ambiante pendant 3 heures 30. Le précipité correspondant au sulfate de la N,N-diméthyl-p-phénylènediamine a été essoré. Après avoir rajouté au filtrat de l'éther diéthylique et de l'eau saturée de chlorure de sodium, on extrait la phase aqueuse par de l'éther diéthylique. On a réuni les phases organiques qu'on a lavé jusqu'à neutralité par de l'eau saturée en chlorure de sodium, on les a séchés sur Na₂SO₄ et on les a concentrés à sec. Après recristallisation par un mélange d'acétate d'éthyle et d'heptane puis séchage à l'air, on a obtenu 15,25g de poudre kaki correspondant à la structure attendue. Les spectres RMN¹H et RMN¹³C du produit obtenu sont conformes à la structure attendue.

### EXEMPLE DE COMPOSITION :

On donne ci-après un exemple concret d'une émulsion huile-dans-eau autobronzante conforme à l'invention :

| Phase A : | |
|---|---|
| - mélange d'alcool cétylstéarylique et d'alcool cétylstéarylique oxyéthyléné (33 OE) 80/20 vendu sous la dénomination « DEHSCONET 390 » par la société TENSIA | 7 % |
| | |
| - mélange de mono et distéarate de glycérol vendu sous la dénomination « CERASYNTH SD » par la société ISP | 2 % |
| - alcool cétylique | 1,5 % |
| - polydiméthylsiloxane vendu sous la dénomination « DC200 Fluid » par la société DOW CORNING | 1,5 % |
| - benzoate d'alcools C₁₂/C₁₅ vendu sous la dénomination | |
| « FINSOLV TN » par la société FINETEX | 15 % |
| - 3-acétamido-1-phényl-4,4-dihydroxypyrazolin-5-one | 1 % |

| Phase B : | |
|---|---|
| - glycérine | 20 % |
| - conservateurs | qs |
| - eau déminéralisée qsp | 100 % |

Cette émulsion a été réalisée selon le mode opératoire suivant : on a amené les phases A et B à 80 °C séparément. Puis on a versé la phase A dans la phase B sous agitation Moritz. On a ensuite homogénéisé le mélange puis on l'a laissé refroidir jusqu'à température ambiante.

## Revendications

1. Composé 4,4-dihydroxypyrazolin-5-one répondant à la formule (I) ci-dessous : 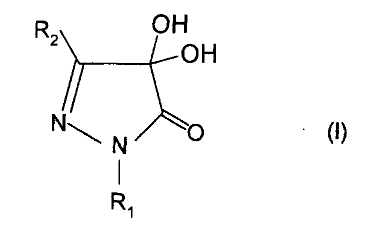 dans laquelle R₁ est choisi parmi :
- un atome d'hydrogène ; un radical alkyle, linéaire ou ramifié, en C₁-C₈,
- un radical -(CH₂)₂-OR' dans lequel R' représente un radical phényle ou naphtyle ;
- un radical -(CH₂)q-R" dans lequel q est égal à 1 ou 2 et R" représente un radical phényle éventuellement substitué par un radical trifluorométhyle ;
- un radical phényle éventuellement substitué par un radical nitro, un radical Cl, un radical alkyle en C₁-C₄ ou un radical alcoxy en C₁-C₄,
et R₂ est choisi parmi :
- un atome d'hydrogène ; un radical alkyle en C₁-C₄, linéaire ou ramifié ; un radical hydroxyalkyle en C₁-C₄ ; un radical méthoxyméthyle ;
- un radical phényle éventuellement substitué par un atome d'halogène, un radical nitro, un radical alkyle en C₁-C₄ ou un radical alcoxy en C₁-C₄ ;
- un radical alcoxy en C₁-C₄ ; un radical trifluorométhyle ; dialkylamino en C₁-C₄ ; carboxy ; méthoxycarbonyle ; éthoxycarbonyle ;
- un radical thiényle, un radical furyle, un radical pyridyle ou un radical pyrazolyle,
- un radical -NHCOR₅ dans lequel R₅ désigne un radical alkyle linéaire ou ramifié en C₁-C₈.

2. Composé selon la revendication 1, **caractérisée par le fait que** R₁ est choisi parmi :
- l'hydrogène et les radicaux méthyle, éthyle, isopropyle, tertiobutyle ou phényle substitué ou non ;
et R₂ est choisi parmi :
- l'hydrogène ou les radicaux méthyle, phényle, méthoxyphényle, méthoxy, éthoxy, carboxy, méthoxycarbonyle, éthoxycarbonyle, acétamido, diméthylamino, diéthylamino, trifluorométhyle, furyle, pyridyle.

3. Composé selon la revendication 2, choisi parmi : la 3-méthyl 4,4-dihydroxypyrazolin-5-one ; la 1,3-diméthyl 4,4-dihydroxypyrazolin-5-one ; la 1-éthyl 3-méthyl 4,4-dihydroxypyrazolin-5-one ; la 1-isopropyl 3-méthyl 4,4-dihydroxypyrazolin-5-one ; la 1-tertbutyl 3-méthyl 4,4-dihydroxypyrazolin-5-one ; la 1-méthyl 3-phényl 4,4-dihydroxypyrazolin-5-one ; la 1-méthyl 3-(3'-méthoxyphényl) 4,4-dihydroxypyrazolin-5-one ; la 3(2'-furyl) 1-méthyl 4,4-dihydroxypyrazolin-5-one ; la 1-méthyl 3-(4'-pyridyl) 4,4-dihydroxypyrazolin-5-one ; la 1-méthyl 3-méthoxy 4,4-dihydroxypyrazolin-5-one ; la 3-éthoxy 1-méthyl 4,4-dihydroxypyrazolin-5-one ; la 3-diméthylamino 1-méthyl 4,4-dihydroxypyrazolin-5-one ; la 3-diéthylamino 1-méthyl 4,4-dihydroxypyrazolin-5-one ; la 3-acétamido 1-méthyl 4,4-dihydroxypyrazolin-5-one ; la 1-phényl 4,4-dihydroxypyrazolin-5-one ; la 1-méthyl 4,4-dihydroxypyrazolin-5-one ; la 1-éthyl 4,4-dihydroxypyrazolin-5-one ; la 1-isopropyl 4,4-dihydroxypyrazolin-5-one ; la 1-tertbutyl 4,4-dihydroxypyrazolin-5-one ; la 3-méthoxy 1-phényl 4,4-dihydroxypyrazolin-5-one ; la 3-éthoxy 1-phényl 4,4-dihydroxypyrazolin-5-one ; la 3-acétamido 1-phényl 4,4-dihydroxypyrazolin-5-one ; la 3-diméthylamino 1-phényl 4,4-dihydroxypyrazolin-5-one ; la 3-diéthylamino 1-phényl 4,4-dihydroxypyrazolin-5-one ; la 1-phényl 3-trifluorométhyl 4,4-dihydroxypyrazolin-5-one ; la 1-méthyl 3-trifluorométhyl 4,4-dihydroxypyrazolin-5-one ; la 1-isopropyl 3-trifluorométhyl 4,4-dihydroxypyrazolin-5-one ; la 1-éthyl 3-trifluorométhyl 4,4-dihydroxypyrazolin-5-one ; la 3-trifluorométhyl 4,4-dihydroxypyrazolin-5-one ; la 3-carboxy 1-phényl 4,4-dihydroxypyrazolin-5-one ; la 3-méthoxycarbonyl 1-phényl 4,4-dihydroxypyrazolin-5-one ; la 3-éthoxycarbonyl 1-phényl 4,4-dihydroxypyrazolin-5-one ; la 3-méthoxy 4,4-dihydroxypyrazolin-5-one ; la 3-éthoxy 4,4-dihydroxypyrazolin-5-one ; la 3-carboxy 1-méthyl 4,4-dihydroxypyrazolin-5-one ; la 3-méthoxycarbonyle 1-méthyl 4,4-dihydroxypyrazolin-5-one ; la 3-éthoxycarbonyle 1-méthyl 4,4-dihydroxypyrazolin-5-one.

4. Composition cosmétique ou dermatologique, **caractérisée par le fait qu'**elle comprend dans un support cosmétiquement acceptable, au moins une 4,4-dihydroxypyrazolin-5-one répondant à la formule (I) tel que défini dans l'une quelconque des revendications 1 à 3.

5. Composition selon la revendications 4, **caractérisée par le fait que** la 4,4-dihydroxypyrazolin-5-one est présente dans la composition à une concentration comprise entre 0,05 et 10 % en poids, par rapport au poids total de la composition.

6. Composition selon l'une quelconque des revendications 4 à 5, **caractérisée par le fait qu'**il s'agit d'une composition cosmétique pour colorer la peau.

7. Composition selon l'une quelconque des revendications 4 à 6, **caractérisée par le fait qu'**il s'agit d'une composition de maquillage destinée à rehausser l'éclat du teint de la peau en particulier du visage, de manière à lui donner un effet bonne mine.

8. Composition selon l'une quelconque des revendications 4 à 7, **caractérisée par le fait qu'**il s'agit d'une composition cosmétique pour colorer les cheveux.

9. Procédé de traitement cosmétique de la peau destiné à la bronzer et/ou la brunir artificiellement, **caractérisé par le fait qu'**il consiste à appliquer sur celle-ci une quantité efficace d'une composition telle que définie à l'une quelconque des revendications 4 à 7, ou d'une 4,4-dihydroxypyrazolin-5-one telle que définie à l'une quelconque des revendications 1 à 3.

10. Utilisation d'une 4,4-dihydroxypyrazolin-5-one telle que définie à l'une quelconque des revendications 1 à 3 dans, ou pour la fabrication de, compositions destinées au bronzage et/ou au brunissage artificiels de la peau.

11. Procédé de maquillage de la peau, en particulier du visage destiné à lui donner un aspect « bonne mine» en rehaussant l'éclat du teint , consistant à appliquer sur la peau une quantité efficace d'une composition cosmétique telle que définie à l'une quelconque des revendications 4 à 7, ou d'une 4,4-dihydroxypyrazolin-5-one telle que définie à l'une quelconque des revendications 1 à 3.

12. Utilisation d'une 4,4-dihydroxypyrazolin-5-one telle que définie à l'une quelconque des revendications 1 à 3 dans, ou pour la fabrication de, compositions de maquillage de la peau, en particulier du visage destiné à lui donner un aspect « bonne mine» en rehaussant l'éclat du teint

13. Procédé de traitement cosmétique des cheveux destiné à les colorer, **caractérisé par le fait qu'**il consiste à appliquer sur les cheveux une quantité efficace d'une composition telle que définie à l'une quelconque des revendications 6 et 7 ou d'une 4,4-dihydroxypyrazolin-5-one telle que définie à l'une quelconque des revendications 1 à 3.

14. Utilisation d'une 4,4-dihydroxypyrazolin-5-one telle que définie dans l'une quelconque des revendications 1 à 3 dans, ou pour la fabrication de, compositions destinées à colorer les cheveux.

15. Procédé de préparation des composés de formule (I) tels que définie dans l'une quelconque des revendications 1 à 3, **caractérisé par le fait qu'**il comprend les étapes suivantes (les significations de R₁ et R₂ sont telles que données dans la revendication 1) :
i) on fait réagir une hydrazine monosubstituée R₁NH-NH₂ avec un β-cétoester de structure : 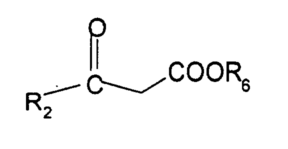 dans laquelle R₆ désigne un radical alkyle linéaire ou ramifié en C₁-C₄, de préférence en milieu alcoolique à une température comprise entre 65 et 85°C, de préférence au reflux du solvant utilisé de façon à obtenir une pyrazolin-5-one ① qu'on fait ensuite réagir avec un composé nitroso-aromatique ② de façon à obtenir la 4-arylimino pyrazolin-5-one correspondante ③ : 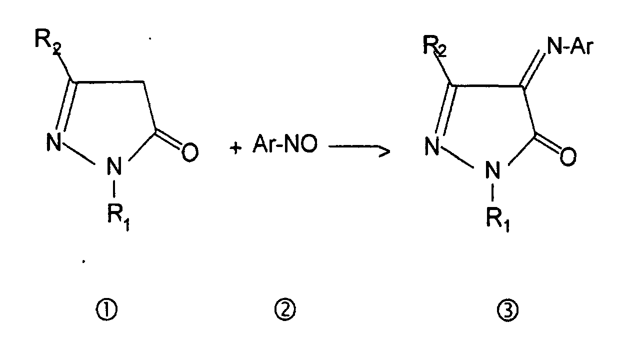 cette réaction étant conduite de préférence dans un alcool inférieur à une température comprise entre 65 °C et 85 °C, au reflux du solvant utilisé, et de préférence en présence d'une base faible de type carbonate ou bicarbonate en quantité catalytique,
(ii) puis on hydrolyse la 4-arylimino pyrazolin-5-one ③, de préférence en milieu acide dilué fort, pour obtenir le dérivé de 4,4-dihydroxypyrazolin-5-one correspondant de formule (I) :

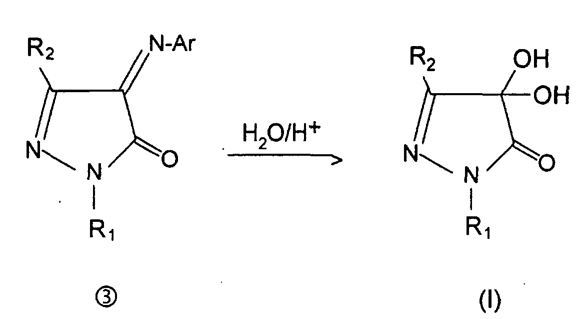

16. Procédé selon la revendication 15, **caractérisé par le fait que** le dérivé nitroso aromatique de l'étape (i) est une p-nitrosodialkylaniline de formule ② suivante : 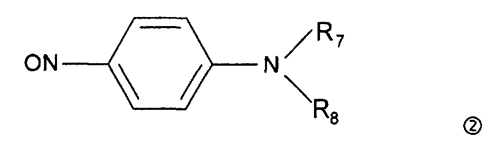 dans laquelle R₇ et R₈ représentent un radical alkyle en C₁-C₄, linéaire ou ramifié.

17. Procédé selon l'une quelconque des revendications 15 à 16, **caractérisé par le fait que** l'hydrolyse acide de l'étape (ii) est réalisée avec de l'acide sulfurique dilué ou de l'acide chlorhydrique aqueux à température ambiante en présence d'un co-solvant de la 4,4-dihydroxypyrazolin-5-one non miscible à l'eau.

18. Procédé selon la revendication 17, **caractérisé par le fait que** ledit co-solvant est un éther choisi parmi l'éther diéthylique et l'éther diisopropylique.

## Patentansprüche

1. 4,4-Dihydroxypyrazolin-5-on der folgenden Formel (I): 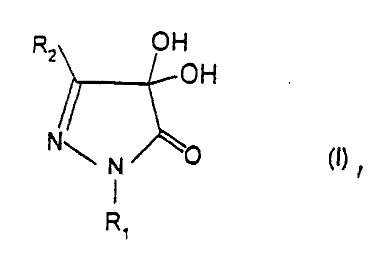 wobei
R₁ ausgewählt ist unter:
- einem Wasserstoffatom; einer geradkettigen oder verzweigten C₁₋₈-Alkylgruppe,
- einer Gruppe -(CH₂)₂-OR', worin R' Phenyl oder Naphthyl bedeutet;
- einer Gruppe -(CH₂)q-R", worin q 1 oder 2 bedeutet und R" eine gegebenenfalls mit einer Trifluormethylgruppe substituierte Phenylgruppe ist;
- einer Phenylgruppe, die gegebenenfalls mit Nitro, Cl, C₁₋₄-Alkyl oder C₁₋₄-Alkoxy substituiert ist; und
R₂ ausgewählt ist unter:
- Wasserstoff; einer geradkettigen oder verzweigten C₁₋₄-Alkylgruppe; einer C₁₋₄-Hydroxyalkylgruppe; Methoxymethyl;
- einer Phenylgruppe, die gegebenenfalls mit einem Halogenatom, Nitro, C₁₋₄-Alkyl oder C₁₋₄-Alkoxy substituiert ist;
- einer C₁₋₄-Alkoxygruppe; Trifluormethyl; C₁₋₄-Dialkylamino; Carboxy; Methoxycarbonyl; Ethoxycarbonyl;
- Thienyl, Furyl, Pyridyl oder Pyrazolyl,
- einer Gruppe -NHCOR₅, wobei Rs eine geradkettige oder verzweigte C₁₋₈-Alkylgruppe bedeutet.

2. Verbindung nach Anspruch 1, **dadurch gekennzeichnet, dass**
R₁ ausgewählt ist unter: Wasserstoff, Methyl, Ethyl, Isopropyl, t-Butyl oder substituierten oder unsubstituierten Phenylgruppen; und
R₂ ausgewählt ist unter Wasserstoff, Methyl, Phenyl, Methoxyphenyl, Methoxy, Ethoxy, Carboxy, Methoxycarbonyl, Ethoxycarbonyl, Acetamido, Dimethylamino, Diethylamino, Trifluormethyl, Furyl und Pyridyl.

3. Verbindung nach Anspruch 2, die ausgewählt ist unter:
3-Methyl-4,4-dihydroxypyrazolin-5-on;
1,3-Dimethyl-4,4-dihydroxypyrazolin-5-on;
1-Ethyl-3-methyl-4,4-dihydroxypyrazolin-5-on;
1-Isopropyl-3-methyl-4,4-dihydroxypyrazolin-5-on;
1-*t*-Butyl-3-methyl-4,4-dihydroxypyrazolin-5-on;
1-Methyl-3-phenyl-4,4-dihydroxypyrazolin-5-on;
1-Methyl-3-(3'-methoxyphenyl)-4,4-dihydroxypyrazolin-5-on;
3-(2'-Furyl)-1-methyl-4,4-dihydroxypyrazolin-5-on;
1-Methyl-3-(4'-pyridyl)-4,4-dihydroxypyrazolin-5-on;
1-Methyl-3-methoxy-4,4-dihydroxypyrazolin-5-on;
3-Ethoxy-1-methyl-4,4-dihydroxypyrazolin-5-on;
3-Dimethylamino-1-methyl-4,4-dihydroxypyrazolin-5-on;
3-Diethylamino-1-methyl-4,4-dihydroxypyrazolin-5-on;
3-Acetamido-1-methyl-4,4-dihydroxypyrazolin-5-on;
1-Phenyl-4,4-dihydroxypyrazolin-5-on;
1-Methyl-4,4-dihydroxypyrazolin-5-on;
1-Ethyl-4,4-dihydroxypyrazolin-5-on;
1-Isopropyl-4,4-dihydroxypyrazolin-5-on;
1-*t*-Butyl-4,4-dihydroxypyrazolin-5-on;
3-Methoxy-1-phenyl-4,4-dihydroxypyrazolin-5-on;
3-Ethoxy-1-phenyl-4,4-dihydroxypyrazolin-5-on;
3-Acetamido-1-phenyl-4,4-dihydroxypyrazolin-5-on;
3-Dimethylamino-1-phenyl-4,4-dihydroxypyrazolin-5-on;
3-Diethylamino-1-phenyl-4,4-dihydroxypyrazolin-5-on;
1-Phenyl-3-trifluormethyl-4,4-dihydroxypyrazolin-5-on;
1-Methyl-3-trifluormethyl-4,4-dihydroxypyrazolin-5-on;
1-Isopropyl-3-trifuormethyl-4,4-dihydroxypyrazolin-5-on;
1-Ethyl-3-trifluormethyl-4,4-dihydroxypyrazolin-5-on;
3-Trifluormethyl-4,4-dihydroxypyrazolin-5-on;
3-Carboxy-1-phenyl-4,4-dihydroxypyrazolin-5-on;
3-Methoxycarbonyl-1-phenyl-4,4-dihydroxypyrazolin-5-on;
3-Ethoxycarbonyl-1-phenyl-4,4-dihydroxypyrazolin-5-on;
3-Methoxy-4,4-dihydroxypyrazolin-5-on;
3-Ethoxy-4,4-dihydroxypyrazolin-5-on;
3-Carboxy-1-methyl-4,4-dihydroxypyrazolin-5-on;
3-Methoxycarbonyl-1-methyl-4,4-dihydroxypyrazolin-5-on;
3-Ethoxycarbonyl-1-methyl-4, 4-dihydroxypyrazolin-5-on.

4. Kosmetische oder dermatologische Zusammensetzung, **dadurch gekennzeichnet, dass** sie in einem kosmetisch akzeptablen Träger mindestens ein 4,4-Dihydroxypyrazolin-5-on der Formel (I) nach einem der Ansprüche 1 bis 3 enthält.

5. Zusammensetzung nach Anspruch 4, **dadurch gekennzeichnet, dass** das 4,4-Dihydroxypyrazolin-5-on in der Zusammensetzung in einer Konzentration von 0,05 bis 10 Gew.-%, bezogen auf das Gesamtgewicht der Zusammensetzung, vorliegt.

6. Zusammensetzung nach einem der Ansprüche 4 bis 5, **dadurch gekennzeichnet, dass** es sich um eine kosmetische Zusammensetzung zum Färben der Haut handelt.

7. Zusammensetzung nach einem der Ansprüche 4 bis 6, **dadurch gekennzeichnet, dass** es sich um eine Zusammensetzung zum Schminken handelt, die den Teint der Haut, insbesondere des Gesichts, strahlender macht, um ihm ein "gutes Aussehen" zu geben.

8. Zusammensetzung nach einem der Ansprüche 4 bis 7, **dadurch gekennzeichnet, dass** es sich um eine kosmetische Zusammensetzung zum Färben der Haare handelt.

9. Verfahren zur kosmetischen Behandlung der Haut, das dazu vorgesehen ist, die Haut zu bräunen und/oder künstlich braun zu färben, **dadurch gekennzeichnet, dass** es darin besteht, auf die Haut eine wirksame Menge einer Zusammensetzung nach einem der Ansprüche 4 bis 7 oder ein 4,4-Dihydroxypyrazolin-5-on nach einem der Ansprüche 1 bis 3 aufzutragen.

10. Verwendung eines 4,4-Dihydroxypyrazolin-5-on nach einem der Ansprüche 1 bis 3 in Zusammensetzungen zum Bräunen und/oder künstlichen Braunfärben der Haut oder zur Herstellung dieser Zusammensetzungen.

11. Verfahren zum Schminken der Haut und insbesondere des Gesichts, das dazu dienen soll, dem Gesicht ein "gutes Aussehen" zu geben, indem der Teint noch strahlender wird, das darin besteht, auf die Haut eine wirksame Menge einer kosmetischen Zusammensetzung nach einem der Ansprüche 4 bis 7 oder ein 4,4-Dihydroxypyrazolin-5-on nach einem der Ansprüche 1 bis 3 aufzutragen.

12. Verwendung eines 4,4-Dihydroxypyrazolin-5-on nach einem der Ansprüche 1 bis 3 in Zusammensetzungen zum Schminken der Haut und insbesondere des Gesichts, um dem Gesicht ein "gutes Aussehen" zu geben, indem der Teint noch strahlender wird.

13. Verfahren zur kosmetischen Behandlung der Haare, das dazu vorgesehen ist, die Haare zu färben, **dadurch gekennzeichnet, dass** es darin besteht, auf die Haare eine wirksame Menge einer Zusammensetzung nach einem der Ansprüche 6 und 7 oder ein 4,4-Dihydroxypyrazolin-5-on nach einem der Ansprüche 1 bis 3 aufzutragen.

14. Verwendung eines 4,4-Dihydroxypyrazolin-5-on nach einem der Ansprüche 1 bis 3 in Zusammensetzungen zum Färben der Haare oder zur Herstellung dieser Zusammensetzungen.

15. Verfahren zur Herstellung von Verbindungen der Formel (I) nach einem der Ansprüche 1 bis 3, **dadurch gekennzeichnet, dass** es die folgenden Schritte umfasst (die Bedeutungen der Gruppen R₁ und R₂ entsprechen den in Anspruch 1 angegebenen Bedeutungen):
(i) ein einfach substituiertes Hydrazin R₁NH-NH₂ wird mit einem β-Ketoester der folgenden Struktur: 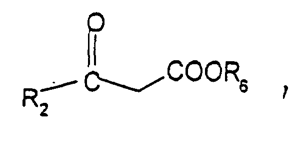 wobei R₆ eine geradkettige oder verzweigte C₁₋₄-Alkylgruppe bedeutet, vorzugsweise in einem alkoholischen Medium bei einer Temperatur im Bereich von 65 bis 85° C und vorzugsweise bei der Rückflußtemperatur des verwendeten Lösungsmittels umgesetzt, um ein Pyrazolin-5-on ① herzustellen, das dann mit einer nitroso-aromatischen Verbindung ② umgesetzt wird, um das entsprechende 4-Aryliminopyrazolin-5-on ③ herzustellen: 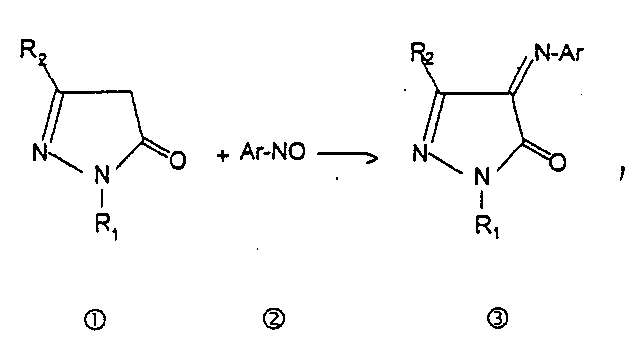 wobei diese Umsetzung vorzugsweise in einem niederen Alkohol bei einer Temperatur im Bereich von 65 bis 85° C bei Rückflußtemperatur des verwendeten Lösungsmittels und vorzugsweise in Gegenwart einer schwachen Base vom Carbonattyp oder Bicarbonattyp in einer katalytischen Menge durchgeführt wird, und
(ii) anschließend das 4-Aryliminopyrazolin-5-on ③ vorzugsweise in einem stark verdünnten sauren Medium hydrolysiert wird, um das entsprechende 4,4-Dihydroxypyrazolin-5-on-Derivat der Formel (I) herzustellen:

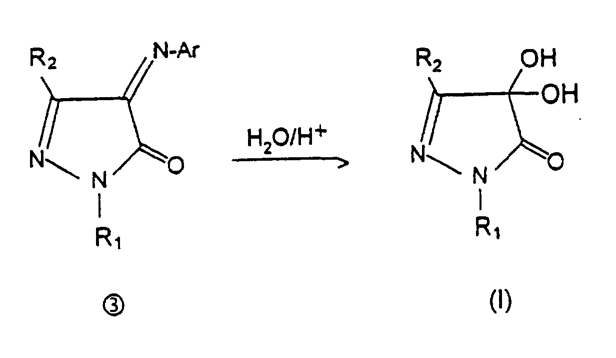

16. Verfahren nach Anspruch 15, **dadurch gekennzeichnet, dass** das nitroso-aromatische Derivat in Schritt (i) ein p-Nitrosodialkylanilin der folgenden Formel ② ist: 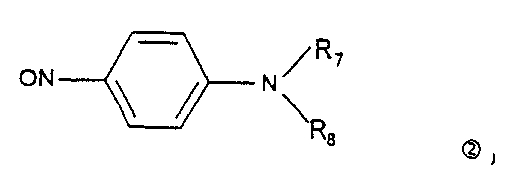 worin R₇ und R₈ eine geradkettige oder verzweigte C₁₋₄-Alkylgruppe bedeuten.

17. Verfahren nach einem der Ansprüche 15 bis 16, **dadurch gekennzeichnet, dass** die saure Hydrolyse in Schritt (ii) mit verdünnter Schwefelsäure oder einer wässrigen Salzsäurelösung bei Raumtemperatur in Gegenwart eines nicht mit Wasser mischbaren Colösungsmittels für das 4,4-Dihydroxypyrazolin-5-on durchgeführt wird.

18. Verfahren nach Anspruche 17, **dadurch gekennzeichnet, dass** das Colösungsmittel ein Ether ist, der unter Diethylether und Diisopropylether ausgewählt ist.

## Claims

1. 4,4-Dihydroxy-5-pyrazolinone compound corresponding to formula (I) below: 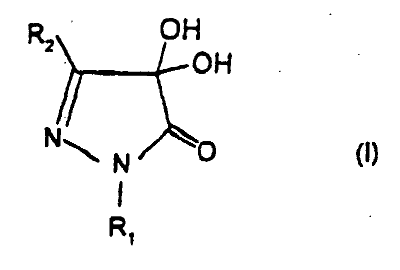 in which R₁ is chosen from:
- a hydrogen atom; a linear or branched C₁-C₈ alkyl radical,
- a radical -(CH₂)₂-OR' in which R' represents a phenyl or naphthyl radical;
- a radical -(CH₂)_{q}-R'' in which q is equal to 1 or 2 and R'' represents a phenyl radical optionally substituted with a trifluoromethyl radical;
- a phenyl radical optionally substituted with a nitro radical, a Cl radical, a C₁-C₄ alkyl radical or a C₁-C₄ alkoxy radical,
and R₂ is chosen from:
- a hydrogen atom; a linear or branched C₁-C₄ alkyl radical; a C₁-C₄ hydroxyalkyl radical; a methoxymethyl radical;
- a phenyl radical optionally substituted with a halogen atom, a nitro radical, a C₁-C₄ alkyl radical or a C₁-C₄ alkoxy radical;
- a C₁-C₄ alkoxy radical; a trifluoromethyl radical; a C₁-C₄ dialkylamino radical; a carboxyl radical; a methoxycarbonyl radical; an ethoxycarbonyl radical;
- a thienyl radical, a furyl radical, a pyridyl radical or a pyrazolyl radical,
- a radical -NHCOR₅ in which R₅ denotes a linear or branched C₁-C₈ alkyl radical.

2. Compound according to Claim 1, **characterized in that** R₁ is chosen from:
- hydrogen and methyl, ethyl, isopropyl, tert-butyl and substituted or unsubstituted phenyl radicals; and R₂ is chosen from:
- hydrogen and methyl, phenyl, methoxyphenyl, methoxy, ethoxy, carboxyl, methoxycarbonyl, ethoxycarbonyl, acetamido, dimethylamino, diethylamino, trifluoromethyl, furyl and pyridyl radicals.

3. Compound according to Claim 2, chosen from: 3-methyl-4,4-dihydroxy-5-pyrazolinone; 1,3-dimethyl-4,4-dihydroxy-5-pyrazolinone; 1-ethyl-3-methyl-4,4-dihydroxy-5-pyrazolinone; 1-isopropyl-3-methyl-4,4-dihydroxy-5-pyrazolinone; 1-tert-butyl-3-methyl-4,4-dihydroxy-5-pyrazolinone; 1-methyl-3-phenyl-4,4-dihydroxy-5-pyrazolinone; 1-methyl-3-(3'-methoxyphenyl)-4,4-dihydroxy-5-pyrazolinone; 3-(2'-furyl)-1-methyl-4,4-dihydroxy-5-pyrazolinone; 1-methyl-3-(4'-pyridyl)-4,4-dihydroxy-5-pyrazolinone; 1-methyl-3-methoxy-4,4-dihydroxy-5-pyrazolinone; 3-ethoxy-1-methyl-4,4-dihydroxy-5-pyrazolinone; 3-dimethylamino-1-methyl-4,4-dihydroxy-5-pyrazolinone; 3-diethylamino-1-methyl-4,4-dihydroxy-5-pyrazolinone; 3-acetamido-1-methyl-4,4-dihydroxy-5-pyrazolinone; 1-phenyl-4,4-dihydroxy-5-pyrazolinone; 1-methyl-4,4-dihydroxy-5-pyrazolinone; 1-ethyl-4,4-dihydroxy-5-pyrazolinone; 1-isopropyl-4,4-dihydroxy-5-pyrazolinone; 1-tert-butyl-4,4-dihydroxy-5-pyrazolinone; 3-methoxy-1-phenyl-4,4-dihydroxy-5-pyrazolinone; 3-ethoxy-1-phenyl-4,4-dihydroxy-5-pyrazolinone; 3-acetamido-1-phenyl-4,4-dihydroxy-5-pyrazolinone; 3-dimethylamino-1-phenyl-4,4-dihydroxy-5-pyrazolinone; 3-diethylamino-1-phenyl-4,4-dihydroxy-5-pyrazolinone; 1-phenyl-3-trifluoromethyl-4,4-dihydroxy-5-pyrazolinone; 1-methyl-3-trifluoromethyl-4,4-dihydroxy-5-pyrazolinone; 2-isopropyl-3-trifluoromethyl-4,4-dihydroxy-5-pyrazolinone; 1-ethyl-3-trifluoromethyl-4,4-dihydroxy-5-pyrazolinone; 3-trifluoromethyl-4,4-dihydroxy-5-pyrazolinone; 3-carboxy-1-phenyl-4,4-dihydroxy-5-pyrazolinone; 3-methoxycarbonyl-1-phenyl-4,4-dihydroxy-5-pyrazolinone; 3-ethoxycarbonyl-1-phenyl-4,4-dihydroxy-5-pyrazolinone; 3-methoxy-4,4-dihydroxy-5-pyrazolinone; 3-ethoxy-4,4-dihydroxy-5-pyrazolinone; 3-carboxy-1-methyl-4,4-dihydroxy-5-pyrazolinone; 3-methoxycarbonyl-1-methyl-4,4-dihydroxy-5-pyrazolinone; 3-ethoxycarbonyl-1-methyl-4,4-dihydroxy-5-pyrazolinone.

4. Cosmetic or dermatological composition, **characterized in that** it comprises, in a cosmetically acceptable support, at least one 4,4-dihydroxy-5-pyrazolinone corresponding to formula (I) as defined in any one of Claims 1 to 3.

5. Composition according to Claim 4, **characterized in that** the 4,4-dihydroxy-5-pyrazolinone is present in the composition at a concentration of between 0.05 and 10% by weight relative to the total weight of the composition.

6. Composition according to either of Claims 4 and 5, **characterized in that** it is a cosmetic composition for colouring the skin.

7. Composition according to any one of Claims 4 to 6, **characterized in that** it is a make-up composition intended to enhance the radiance of the complexion of the skin, in particular of the face, so as to give it a healthy appearance.

8. Composition according to any one of Claims 4 to 7, **characterized in that** it is a cosmetic composition for dyeing the hair.

9. Cosmetic treatment process for the skin which is intended to artificially tan and/or brown it, **characterized in that** it consists in applying an effective amount of a composition as defined in any one of Claims 4 to 7 or a 4,4-dihydroxy-5-pyrazolinone as defined in any one of Claims 1 to 3 to the skin.

10. Use of a 4,4-dihydroxy-5-pyrazolinone as defined in any one of Claims 1 to 3 in, or for the manufacture of, compositions intended to artificially tan and/or brown the skin.

11. Make-up process for the skin, in particular the face, which is intended to give it a healthy appearance by enhancing the radiance of the complexion, this process consisting in applying an effective amount of a cosmetic composition as defined in any one of Claims 4 to 7 or a 4,4-dihydroxy-5-pyrazolinone as defined in any one of Claims 1 to 3 to the skin.

12. Use of a 4,4-dihydroxy-5-pyrazolinone as defined in any one of Claims 1 to 3 in, or for the manufacture of, make-up compositions for the skin, in particular the face, which is intended to give it a healthy appearance by enhancing the radiance of the complexion.

13. Cosmetic treatment process for the hair which is intended to dye it, **characterized in that** it consists in applying an effective amount of a composition as defined in either of Claims 6 and 7 or a 4,4-dihydroxy-5-pyrazolinone as defined in any one of Claims 1 to 3 to the hair.

14. Use of a 4,4-dihydroxy-5-pyrazolinone as defined in any one of Claims 1 to 3 in, or for the manufacture of, compositions intended to dye the hair.

15. Process for the preparation of the compounds of formula (I) as defined in any one of Claims 1 to 3, **characterized in that** it comprises the following steps (the meanings of R₁ and R₂ are as given in Claim 1) :
(i) a monosubstituted hydrazine R₁NH-NH₂ is reacted with a β-keto ester of structure: 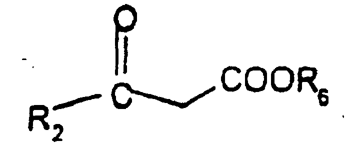 in which R₆ denotes a linear or branched C₁-C₄ alkyl radical, preferably in alcoholic medium at a temperature of between 65 and 85°C, preferably at the reflux point of the solvent used, so as to obtain a 5-pyrazolinone (1), which is then reacted with an aromatic nitroso compound (2) so as to obtain the corresponding 4-arylimino-5-pyrazolinone (3): 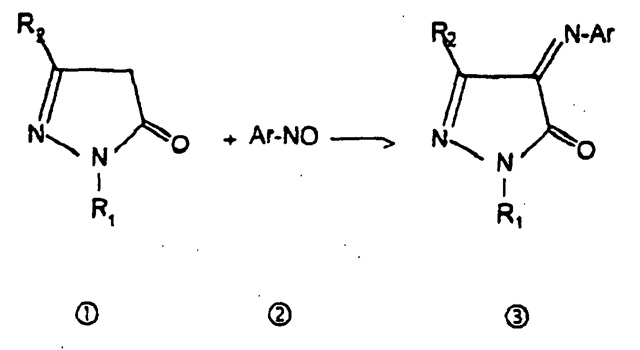 this reaction preferably being carried out in a lower alcohol at a temperature of between 65°C and 85°C, at the reflux point of the solvent used, and preferably in the presence of a catalytic amount of a weak base of carbonate or bicarbonate type,
(ii) the 4-arylimino-5-pyrazolinone (3) is then hydrolysed, preferably in dilute strong acid medium, in order to obtain the corresponding 4,4-dihydroxy-5-pyrazolinone derivative of formula (I):

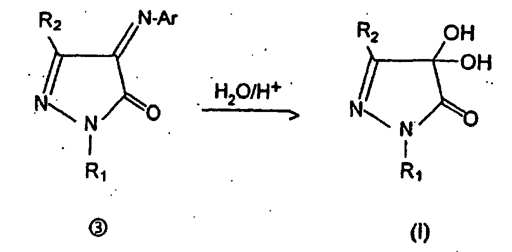

16. Process according to Claim 15, **characterized in that** the aromatic nitroso derivative from step (i) is a p-nitrosodialkylaniline of formula (2) below: 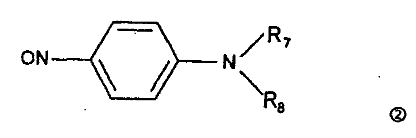 in which R₇ and R₈ represent a linear or branched C₁-C₄ alkyl radical.

17. Process according to either of Claims 15 and 16, **characterized in that** the acid hydrolysis in step (ii) is carried out with dilute sulphuric acid or aqueous hydrochloric acid at room temperature in the presence of a co-solvent for the 4,4-dihydroxy-5-pyrazolinone, which is immiscible with water.

18. Process according to Claim 17, **characterized in that** the said co-solvent is an ether chosen from diethyl ether and diisopropyl ether.
